# NEUE EUROPÄISCHE PATENTSCHRIFT

(11) **EP 1 455 215 B2**
(45) Veröffentlichungstag und Bekanntmachung des Hinweises auf die Entscheidung über den Einspruch: **11.01.2012**
(45) Hinweis auf die Patenterteilung: 11.06.2008
(21) Anmeldenummer: 04003065.2
(22) Anmeldetag: 11.02.2004
(51) Int. Cl.: G02B 21/06, A61B 3/13

(54) **Beleuchtungseinrichtung für ein Mikroskop**
Microscope illuminating apparatus
Dispositif d'illumination pour un microscope

(30) Priorität: 06.03.2003 DE 10311000
(43) Veröffentlichungstag der Anmeldung: 08.09.2004
(73) Patentinhaber: Leica Microsystems (Schweiz) AG, 9435 Heerbrugg (CH)
(72) Erfinder: Sander, Ulrich Dr., 9445 Rebstein (CH)
(74) Vertreter: Kudlek & Grunert Patentanwälte

(56) Entgegenhaltungen:
- EP-A- 1 109 046
- DE-A- 3 327 672
- DE-A- 4 028 605
- DE-A- 4 331 635
- DE-A- 19 611 044
- GB-A- 1 277 979
- US-A- 5 446 582
- US-A1- 2001 040 726
- PATENT ABSTRACTS OF JAPAN Bd. 1998, Nr. 10, 31. August 1998 (1998-08-31) & JP 10 133122 A (NIKON CORP), 22. Mai 1998 (1998-05-22) & Gefunden im Internet: URL:http://www.ipdl.ncipi.go.jp/homepg_e.i pdl> [gefunden am 2004-11-25]

## Beschreibung

Die vorliegende Erfindung betrifft eine Beleuchtungseinrichtung für ein Operationsmikroskop nach dem Oberbegriff des Patentanspruchs 1.

Beleuchtungseinrichtungen für Operationsmikroskope verwenden in der Regel einen Beleuchtungsstrahlengang, der bezüglich des Beobachtungsstrahlengangs einen Winkel im Bereich von etwa 6° aufweist (sogenannte 6°-Beleuchtung). Hierdurch vermeidet man eine unerwünschte Schattenbildung, welche bei größeren Winkeln zwischen Beobachtungsstrahlengang und Beleuchtungsstrahlengang auftreten würde.

Augenchirurgische Operationen stellen weitere besondere Anforderungen an die Beleuchtung eines Mikroskops. Zunächst erhält man eine ausreichende Plastizität des Bildes bei einem Beleuchtungswinkel von wiederum etwa 6°. Es ist jedoch für bestimmte augenchirurgische Beobachtungen oder Eingriffe notwenig, den so genannten Rotreflex zu erzeugen. Hierbei leuchtet die Pupille des operierten Auges durch von der Netzhaut zurückgestreutes Licht rötlich auf. Diese Beleuchtungsart ist beispielsweise bei Katarakt-Operationen von großer Bedeutung, da hierbei Gewebereste im Gegenlicht des Rotreflexes besonders gut zu erkennen sind. Die Rotreflexerzeugung benötigt kleinere Winkel zwischen dem Beobachtungsstrahlengang und dem Beleuchtungsstrahlengang, wobei hier Winkel im Bereich von 0°- 2° bevorzugt werden (sogenannte 2°-Beleuchtung).

Operationsmikroskope, welche mit zwei Paaren von stereoskopischen Beobachtungsstrahlengängen für einen Hauptoperateur bzw. einen Mitbeobachter ausgebildet sind, weisen oftmals insofern einen Mangel auf, als der Rotreflex zwar für den Hauptoperateur sehr gut, für den Mitbeobachter aber nur unzulänglich zu beobachten ist. Dieser erhält in Abhängigkeit von seiner Positionierung, entweder rechts oder links vom Hauptoperateur nur in einem seiner beiden Beobachtungskanäle einen guten Rotreflex. Dies wirkt sich störend auf die stereoskopische Beobachtung aus.

Aus der DE 040 28 605 ist eine Beleuchtungseinrichtung für ein Operationsmikroskop mit einem Beleuchtungssystem, das außerhalb der optischen Achse des Mikroskopobjektivs angeordnet ist, und das Operationsgebiet parallel zur Objektivachse durch das Mikroskopobjektiv hindurch beleuchtet, und einem Umlenkelement auf der objektabgewandten Seite des Mikroskopobjektivs, welches das Operationsgebiet mit einem Bruchteil des Beleuchtungslichtes entlang der Objektivachse beleuchtet, bekannt. Diese Beleuchtungseinrichtung zeichnet sich dadurch aus, dass das Beleuchtungssystem objektivseitig mit einem Reflexionselement ausgestattet ist, welches das Beleuchtungslicht parallel zur Objektivachse zum Mikroskopobjektiv hin reflektiert, und dass das Umlenkelement das Operationsgebiet unter einem Neigungswinkel gegenüber der Objektivachse beleuchtet, der kleiner ist als der Neigungswinkel, unter dem das Reflexionselement das Operationsgebiet beleuchtet. Der größere Neigungswinkel beträgt hier bevorzugt 6°, der kleinere ist von 0° bis 6° variierbar. Als nachteilig bei dieser Konstruktion wird empfunden, dass die von dem Umlenkelement reflektierte Strahlung Randstrahlung der Beleuchtungspupille des Beleuchtungssystems ist, so dass bei einer achsnahen Beleuchtung, beispielsweise unter einem Winkel von 2° zum Beobachtungsstrahlengang, eine relativ inhomogene und vignettierte Ausleuchtung des Leuchtfeldes zu beobachten ist.

Weitere Beleuchtungseinrichtungen für Operationsmikroskope sind aus der DE 196 50 773 A1 und der EP 1 109 046 A1 bekannt. Auch diese Beleuchtungseinrichtungen verwenden für die achsnahe Beleuchtung Randstrahlen der Beleuchtungspupille der Beleuchtungseinrichtung, so dass es auch hier zu den genannten Nachteilen kommt.

Als weiterer Nachteil beim Stand der Technik wird angesehen, dass die dort beschriebenen Operationsmikroskope relativ hoch bauen, da die 2°-Beleuchtung und die 6°-Beleuchtung übereinander angeordnet sind.

Die JP 10 133122 A zeigt ein Operationsmikroskop zum Beobachten einer Augenoperation. Das Operationsmikroskop weist eine gattungsgemäße Beleuchtungseinrichtung auf, bei der unterschiedlich ausgestaltete optische Elemente in einen Beleuchtungsstrahlengang einschiebbar sind, um unterschiedliche Beleuchtungswinkel zu realisieren.

Die Erfindung strebt an, eine Beleuchtungseinrichtung für ein Mikroskop zur Verfügung zu stellen, welche gegenüber herkömmlichen Einrichtungen dieser Art eine homogenere und vignettierungsfreiere Ausleuchtung des Leuchtfeldes ermöglicht. Gleichzeitig wird angestrebt, eine möglichst klein bauende Beleuchtungseinrichtung zur Verfügung zu stellen, so dass die Bauhöhe eines Mikroskops nicht in unerwünschter Weise vergrößert wird.

Dieses Ziel wird erreicht durch eine Beleuchtungseinrichtung mit den Merkmalen des Patentanspruchs 1 sowie ein Operationsmikroskop mit den Merkmalen des Patentanspruchs 3.

Erfindungsgemäß ist eine besonders homogene und vignettierungsfreie Ausleuchtung des Leuchtfeldes des Mikroskops gewährleistet, da durch die Verwendung von Umlenkelementen (zur Umlenkung von Licht aus einem Beleuchtungssystem auf ein zu beobachtendes Objekt), welche als physikalische Strahlenteiler ausgebildet sind, im wesentlichen der volle Querschnitt der Beleuchtungspupille sämtliche Spiegelelemente bzw. Umlenkelemente durchsetzt bzw. trifft. Bei den Beleuchtungseinrichtungen gemäß dem Stand der Technik wurden lediglich kleine, vollständig reflektierende Segmente der Umlenkelemente zur Umlenkung der Randbereiche der Beleuchtungspupillen verwendet. Bei den Strahlenteilern gemäß dem Stand der Technik handelt es sich somit um geometrische Strahlenteiler. Mit der erfindungsgemäßen Umlenkeinrichtung ist es insbesondere möglich, die einzelnen Umlenkelemente entlang einer einzigen optischen Achse zu positionieren, wodurch die Bauhöhe eines Mikroskops vorteilhaft vermindert werden kann.

Vorteilhafte Ausgestaltungen der erfindungsgemäßen Beleuchtungseinrichtung sowie des erfindungsgemäßen Mikroskops sind Gegenstand der Unteransprüche.

Erfindungsgemäß weist die Umlenkeinrichtung drei Umlenkelemente auf. Die Ausgestaltung mit drei Umlenkelementen ermöglicht beispielsweise bei Operationsmikroskopen eine 6°-Beleuchtung, +2°-Beleuchtung und -2°-Beleuchtung.

Gemäß einer besonders bevorzugten Ausführungsform der Erfindung sind zwei Umlenkelemente derart angeordnet, dass eine gleichzeitige +2°- und -2°-Beleuchtung möglich ist. Diese Beleuchtungsmöglichkeit erweist sich insbesondere bei den Rotreflex einsetzenden operativen Techniken als vorteilhaft, bei denen es zu einer Verrollung des Auges kommen kann. Als Beispiel sei hier die Phakoemulsifikation genannt. Bei gleichzeitiger +2°- und -2°-Beleuchtung ist gewährleistet, dass der Operateur während der Arbeit keine Einstellungen am Mikroskop verändern muss. Es ist auch eine gleichzeitige +2°-, -2°- und 6°-Beleuchtung möglich, sowie auch beliebige andere Kombinationen, welche beispielsweise durch Verwendung von entsprechend positionierbaren Blenden eingestellt werden können.

Erfindungsgemäß ist wenigstens eines der Umlenkelemente wenigstens teilweise vollverspiegelt ausgebildet. Mittels dieser Maßnahme kann Licht, welches auf die vollverspiegelten Bereiche auftrifft, vollständig auf das zu beobachtende Objekt gelenkt werden, wodurch die Beleuchtungsstärke vorteilhaft beeinflussbar ist. Ferner können auf diese Weise Überlappungen zwischen den Beobachtungsstrahlengängen des Mikroskops und den vollverspiegelten Bereichen in einfacher Weise konstruiert werden. Derartige Überlappungen sind für bestimmte Anwendungen im Rahmen der Augenchirurgie, beispielsweise zur Bereitstellung bzw. Einstellung des Rotreflexes notwendig.

Erfindungsgemäß ist die Umlenkeinrichtung als einheitlicher bzw. einstückiger Prismenblock ausgebildet. Bei einem derartigen einheitlichen Prismenblock ist die bei der Mikroskopmontage notwendige Justierarbeit auf ein Minimum begrenzbar. Ein derartiger Prismenblock erweist sich ferner im Mikroskopbetrieb als sehr robust.

Gemäß einer bevorzugten Ausgestaltung des erfindungsgemäßen Mikroskops ist dieses als Stereomikroskop ausgebildet. Hierbei wird besonders bevorzugt, dass das Stereomikroskop zwei Beobachtungsstrahlengänge für einen Hauptoperateur und zwei weitere Beobachtungsstrahlengänge für einen Assistenten aufweist. Insbesondre bei einem derartigen Stereomikroskop mit Beobachtungsstrahlengängen für einen Hauptoperateur und einen Assistenten lassen sich im Vergleich zu herkömmlichen Mikroskopen dieser Art sowohl für den Hauptoperateur als auch den Assistenten Rotreflexeigenschaften in gewünschter Weise einstellen. Die Ausnutzung im wesentlichen der gesamten Beleuchtungspupille führt insgesamt dazu, dass Vignettierungen weitgehend vermieden werden können, und sowohl für den Hauptbeobachter als auch den Assistenten ein homogenes Leuchtfeld bereitgestellt werden kann.

Zweckmäßigerweise weist das erfindungsgemäße Mikroskop Blenden auf, mit denen die Beleuchtungsstrahlengänge wahlweise eingeschaltet oder abgeschaltet werden können. Hierdurch ist es beispielsweise möglich, eine 0°-Beleuchtung im wesentlichen abzustellen, so dass die Entstehung des Rotreflexes bei Bedarf auch vermieden werden kann.

Ferner ist es gemäß einer besonders bevorzugten Ausgestaltung des erfindungsgemäßen Mikroskops vorgesehen, dass die Umlenkeinrichtung bezüglich der optischen Achse des Mikroskop-Hauptobjektivs querverschiebbar ist. Hierbei ist insbesondere eine Querverschiebbarkeit senkrecht zur optischen Achse des Hauptobjektivs vorgesehen. Hierdurch ergeben sich weitere Variationsmöglichkeiten zur Einstellung von Beleuchtungswinkeln und/oder Überlappungen zwischen vollverspiegelten Bereichen der Umlenkelemente und Beobachtungsstrahlengängen, wodurch die Rotreflexausbildung beeinflussbar ist.

Die Erfindung wird nun anhand der beigefügten Zeichnungen weiter beschrieben. In dieser zeigt
Figur 1 eine schematisch vereinfachte, seitliche Schnittansicht einer bevorzugten Ausführungsform des erfindungsgemäßen Mikroskops, welches eine bevorzugte Ausführungsform der erfindungsgemäßen Beleuchtungseinrichtung aufweist,
Figur 2 eine schematische Ansicht einer ersten bevorzugten Ausführungsform der erfindungsgemäß eingesetzten Umlenkeinrichtung in Richtung des Pfeils P in Figur 1,
Figur 3 eine weitere bevorzugte Ausführungsform der erfindungsgemäß eingesetzten Umlenkeinrichtung in Richtung des Pfeils P,

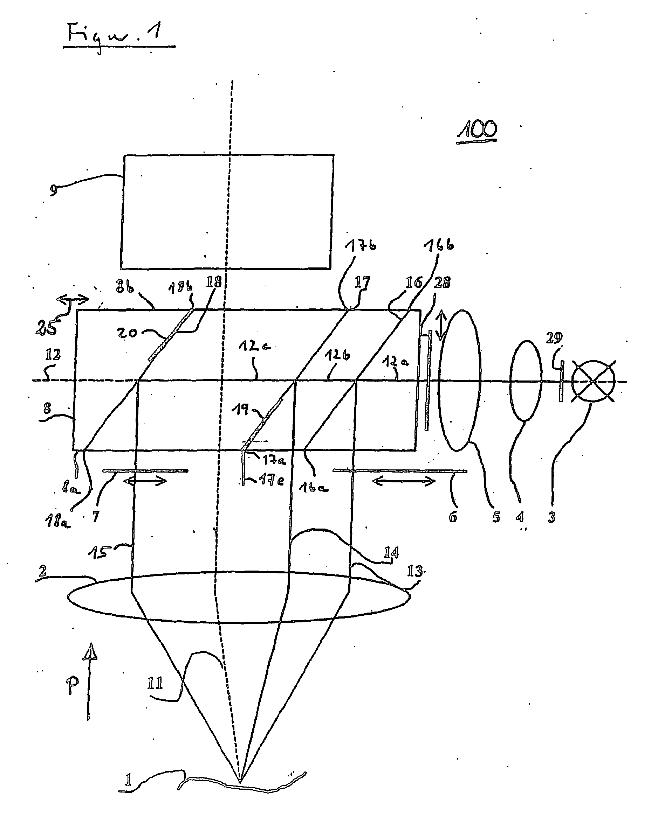

In Figur 1 ist eine bevorzugte Ausführungsform des erfindungsgemäßen Mikroskops insgesamt mit 100 bezeichnet.

Das Mikroskop 100 weist ein Hauptobjektiv 2 und ein insbesondere als Zoom-System ausgebildetes Vergrößerungssystem 9 auf. Die Achse des aus Hauptobjektiv 2 und Vergrößerungssystem 9 gebildeten optischen Gesamtsystems ist mit 11 bezeichnet. Die Beobachtungskanäle des Mikroskops 100 verlaufen parallel zu dieser Achse 11. Wie man aus Figur 1 erkennt, weist diese Achse 11 im Hauptobjektiv 2 einen durch die asymmetrische Positionierung des Vergrößerungssystems 9 bezüglich des Hauptobjektivs 2 verursachten Knick auf. Diese asymmetrische Positionierung von Hauptobjektiv 2 und Vergrößerungssystem 9 erweist sich für bestimmte Anwendungen als vorteilhaft. So würde es bei ausreichend großer Beleuchtungspupille der weiter unten erläuterten 6°-Beleuchtung (Teilstrahlenbündel 13) bei mittiger bzw. symmetrischer Anordnung des Hauptobjektivs 2 bezüglich des Vergrößerungssystems 9 zu einem Abschneiden von Randstrahlung kommen, da in diesem Fall die in der Figur 1 rechte Seite des Hauptobjektivs in die Beleuchtungspupille des Teilstrahlenbündels 13 fallen würde.
Licht zur Beleuchtung eines zu beobachtenden Objektes 1 gelangt von einer Lichtquelle 3 über eine als Prismenblock 8 ausgebildete Umlenkeinrichtung auf das Objekt 1. Zwischen der Lichtquelle 3 und der Umlenkeinrichtung 8 sind beispielhaft zwei Linsen 4, 5 sowie zwei Blenden 28, 29 vorgesehen. Als Lichtquelle 3 sind alle üblichen Lichtquellen, insbesondere kohärente und/oder inkohärente Lichtquellen, wie beispielsweise Glühbirnen, Faserbeleuchtungen, Entladungslampen, Laser usw. einsetzbar.

Der Prismenblock 8 weist insgesamt 3 als verspiegelte Flächen ausgebildete Umlenkelemente 16, 17, 18 auf.

Das Umlenkelement 16 ist vollständig, das Umlenkelement 17 wenigstens teilweise als physikalischer Strahlenteiler ausgebildet. Das heißt, der in der Darstellung der Figur 1 von rechts auf die Umlenkelemente 16, 17 auftreffende Bündelquerschnitt des (schematisch dargestellten) Lichtbündels 12a aus der Lichtquelle 3 bleibt unverändert. Die Aufteilung des Lichtbündels 12a erfolgt gleichmäßig über den gesamten Querschnitt der Umlenkelemente 16, 17. Man erkennt, dass das entlang der Beleuchtungsachse 12 der Umlenkeinrichtung 8 einfallende Lichtbündel 12a am Umlenkelement 16 in ein erstes Teilstrahlenbündel 13, welches reflektiert wird, und ein zweites Teilstrahlenbündel 12b, welches transmittiert wird, aufgeteilt wird. Teilstrahlenbündel 13 stellt nach Durchgang durch das Hauptobjektiv 2 eine 6°-Beleuchtung für das Objekt 1 zur Verfügung.

Das an dem Umlenkelement 16 transmittierte Teilstrahlenbündel 12b wird an dem zweiten Umlenkelement 17 ebenfalls wiederum teilweise reflektiert und transmittiert. Das reflektierte Teilstrahlenbündel ist mit 14, das transmittierte Teilstrahlenbündel mit 12c bezeichnet. Das reflektierte Teilstrahlenbündel 14 verläuft zunächst im wesentlichen parallel zu dem Teilstrahlenbündel 13. Das Teilstrahlenbündel 14 stellt nach Durchgang durch das Hauptobjektiv 2 eine +2°-Beleuchtung des Objekts 1 zur Verfügung.

Das an dem Umlenkelement 17 transmittierte Teilstrahlenbündel 12c trifft anschließend auf das dritte Umlenkelement 18, welches zweckmäßigerweise vollverspiegelt ausgebildet ist. Das an dem Umlenkelement 18 reflektierte, mit 15 bezeichnetet Teilstrahlenbündel trifft, nach Durchgang durch das Hauptobjektiv 2, ebenfalls auf das Objekt 1. Das Teilstrahlenbündel 15 stellt eine -2°-Beleuchtung des Objekts 1 dar. Mittels weiterhin vorgesehener Blenden 6, 7 können die Teilstrahlenbündel 13, 14, 15 ausgeschaltet oder teilweise abgeblendet werden. Hiermit können beispielsweise bei der Beobachtung eines Auges unter Umständen störende Kornearreflexe vermieden, oder auch der Kontrast des Rotreflexes verbessert werden.

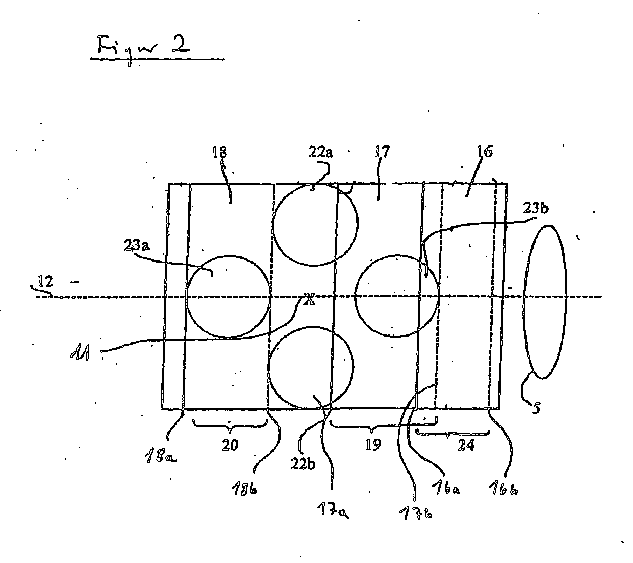

Die Anordnung der bereits erwähnten Beobachtungsstrahlengänge des Mikroskops bezüglich der Umlenkelemente bzw. Reflexionsflächen 16, 17, 18 wird aus Figur 2 deutlich. Figur 2 stellt eine Projektion der Umlenkelemente auf die Unterseite 8a der Umlenkeinrichtung 8 dar. Die jeweiligen Unterkanten 16a, 17a, 18a der Umlenkelemente 16, 17, 18, welche tatsächlich in dieser Unterseite 8a verlaufen, sind mit durchgezogenen Linien dargestellt. Die Oberkanten 16b, 17b, 18b der Umlenkelemente 16, 17, 18, welche in der Oberseite 8b verlaufen, sind mit gestrichelten Linien dargestellt.

In Figur 2 erkennt man die Beobachtungsstrahlengänge des Mikroskops, wobei zwei Beobachtungsstrahlengänge 22a, 22b für ein Hauptbeobachter bzw. -operateur und zwei Beobachtungsstrahlengänge 23a, 23b für einen Mitbeobachter bzw. Assistenten vorgesehen sind.

Man erkennt, dass die Mitbeobachterstrahlengänge 23a, 23b vollständig, und die Hauptbeobachterstrahlengänge 22a, 22b teilweise von den Projektionen der Umlenkelemente bzw. Spiegelflächen 16, 17, 18 überlagert werden. Die vollständig oder teilweise verspiegelten Bereiche der Umlenkelemente 16, 17, 18 sind in Figur 2 mit geschweiften Klammern veranschaulicht und mit 24, 19, bzw. 20 bezeichnet. Es sei noch einmal darauf hingewiesen, dass in Ausführungsbeispiel gemäß Figur 2 die verspiegelten Bereiche im wesentlichen die gesamte Fläche der Umlenkelemente 16, 17, 18 überdecken. Bei beispielsweise vollständiger Verspiegelung der Umlenkelemente 17, 18 sind daher die Beobachtungsstrahlengänge 23a, 23b blockiert, so dass ein derart ausgebildetes Mikroskop lediglich für die stereoskopische Beobachtung durch einen Hauptbeobachter geeignet ist. (Benutzung der Beobachtungskanäle 22a, 22b).

Bei teilverspiegelter Ausbildung der Umlenkelemente 17, 18 ist hingegen eine Beobachtung des Objekts 1 auch durch die Beobachtungskanäle 23a, 23b möglich.

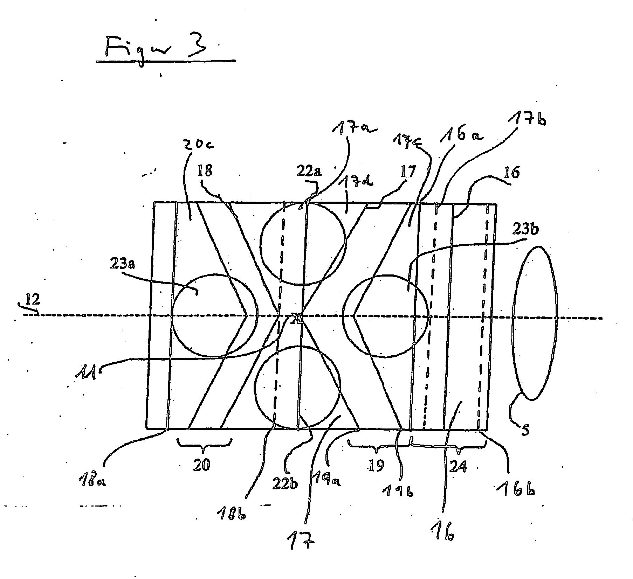

Zur Optimierung der Lichtausbeute in den dargestellten Beobachtungsstrahlengängen wird bevorzugt, die Umlenkelemente 17, 18 teilweise vollverspiegelt auszubilden, wobei nicht vollverspiegelte Bereiche der Umlenkelemente 17, 18 entweder halbdurchlässig bzw. teilverspiegelt oder transparent ausgebildet sein können. Dieser Sachverhalt wird nun unter Bezugnahme auf Figur 3 näher erläutert.

Die Umlenkelemente 17, 18, wie sie in Figur 3 dargestellt sind, zeichnen sich dadurch aus, dass sie nur teilweise vollverspiegelt ausgebildet sind. Die Unterkanten und Oberkanten der Umlenkelemente 17, 18 sind wiederum mit 17a bzw. 17b und 18a bzw. 18b bezeichnet. Zwischen den Kanten 17a, 17b des Umlenkelements 17 erkennt man einen in der dargestellten Projektion pfeilförmig ausgebildeten Bereich 19, welcher eine untere Kante 19a und eine obere Kante 19b aufweist. Bei diesem Bereich 19 handelt es sich wiederum um den vollverspiegelten Bereich des Umlenkelements 17. Der Bereich rechts neben der Oberkante 19b, hier als 17c bezeichnet, sowie der Bereich links neben der Unterkante 19a, hier als Bereich 17d bezeichnet, ist transparent ausgebildet, so dass die entsprechenden Überlappungsbereiche zwischen den Beobachtungskanälen 23b bzw. 22a, 22b und dem vollverspiegelten Bereich 19 verkleinert sind. Somit ist eine im wesentlichen ungehinderte Beobachtung durch die Beobachtungskanäle 22a, 22b und 23b gewährleistet.

Umlenkelement 18 ist in analoger Weise ausgebildet, wobei hier der vollständig verspiegelte Bereich wiederum mit 20 bezeichnet ist. Im Vergleich zur Ausführungsform gemäß Figur 2 erkennt man, dass der Überlappungsbereich zwischen dem Vollverspiegelungsbereich 20 und dem Beobachtungskanal 23a stark vermindert ist, so dass insgesamt eine stereoskopische Beobachtung für einen Mitbeobachter unter Verwendung der Strahlengänge 23a, 23b zur Verfügung gestellt ist. Auch die Überlappungsbereiche zwischen dem vollverspiegelten Bereich 19 und den Beobachtungsstrahlengängen 22a, 22b sind gegenüber der Ausführung gemäß Figur 2 vermindert.

Die vollverspiegelten Bereiche 19, 20 gemäß Figur 3, sind in Figur 1 schematisch als fettgedruckte Linien auf den Umlenkelementen 17, 18 eingezeichnet.

Es sei angemerkt, dass eine Überlappung der verspiegelten Bereiche 19, 20 mit den Beobachtungsstrahlengängen 22a, 22b, 23a, 23b zur Erzeugung des bei bestimmten Anwendungen gewünschten Rotreflexes in gewissem Umfang notwendig ist.

Durch entsprechende Dimensionierung bzw. Ausbildung der vollverspiegelten Bereiche 19, 20 auf den Umlenkelementen 17, 18 ist dieser Rotreflex, beispielsweise bezüglich Intensität und Kontrast, optimierbar.

Wie bereits erwähnt, dient die +/-2°-Beleuchtung dazu, den Rotreflex optimal zu beobachten. Eine Umschaltung der Beleuchtung von einer +2°-Beleuchtung auf eine -2°-Beleuchtung soll insbesondere dazu dienen, den Rotreflex dann zu verbessern, wenn das Patientenauge beispielsweise bei der Phako-Emulsifikation vom Operateur weg verrollt wird. Bei herkömmlichen Mikroskopen war es notwendig, einen +/-2°-Spiegel aktiv in die entsprechende Position durch Betätigung eines Drehknopfes einzustellen. Erfindungsgemäß ist eine gleichzeitige +2- und -2°-Beleuchtung in einfacher Weise bereitstellbar, so dass dem Operateur keine derartigen, von der eigentlichen Operation ablenkenden Tätigkeiten zugemutet werden müssen.

Durch die in einfacher Weise variierbare Überlappung der Vollverspiegelungsbereiche auf den Umlenkelementen mit den Beobachtungsstrahlenkanälen sind sowohl für den Hauptoperateur als auch den Assistent jeweils in beiden Beobachtungsstrahlenkanälen optimale Rotreflexe erzielbar. Die +2°-Beleuchtung dient in der Regel zur Beobachtung des Rotreflexes bei zentriertem Patientenauge. Die - 2°-Beleuchtung ist insbesondere bei dezentriertem Patientenauge vorteilhaft einsetzbar.

Die erfindungsgemäße Beleuchtungseinrichtung zeichnet sich durch eine sehr einfache Handhabbarkeit bei der praktischen Anwendung aus. Aufgrund der Zusammenfassung der Umlenkelemente zu einem einzigen Prismenblock können Justierarbeiten zur Schaffung optimaler Abstände zwischen den Umlenkelementen 16, 17, 18 vollständig bzw. weitgehend vermieden werden. Bei der Ausführungsform gemäß Figur 1 ist es möglich, den Prismenblock 8 entlang der Achse 12 zu verschieben, wie dies mittels des Doppelpfeils 25, anschaulich dargestellt ist.

Es ist ferner möglich, die Umlenkelemente 16, 17, 18 teilweise geschwärzt auszubilden. Mit dieser Maßnahme können unerwünschte Reflexe wirksam unterdrückt werden. Es ist ebenfalls möglich, beispielsweise an der Unterseite der Umlenkeinrichtung 8 absorbierende Elemente, insbesondere Bleche vorzusehen, mit denen ebenfalls unerwünschte Reflexe aufgrund von Mehrfachspiegelungen vermieden werden können. Ein derartiges Blech ist in Figur 1 schematisch dargestellt und mit 17e bezeichnet.

Mit der Schwärzung der Flächen können insbesondere auch interne Reflexe vermieden werden. Mittels der vorgesehenen Elemente, beispielsweise dem Blech 17e können unerwünschte Reflexe am Hauptobjektiv vermieden werden.

Der Prismenblock gemäß Figur 1 ist insbesondere durch Verwendung zweier im wesentlichen identischer Prismenblöcke, welche rotationssymmetrisch zueinander verdreht werden, herstellbar, wobei zwischen die rotationssymmetrisch versetzten Prismen ein Parallelogramm-Block einzusetzen ist.
Es sei angemerkt, dass in den dargestellten Ausführungsformen des Mikroskops die Beobachtungskanäle im wesentlichen symmetrisch um die Beobachtungsachse 11 angeordnet sind. Diese Beobachtungsachse entspricht nicht notwendigerweise der Mittelachse bzw. optischen Achse des Hauptobjektivs 2.

### Bezugszeichenliste

- 1: Objekt
- 2: Hauptobjektiv
- 3: Lichtquelle
- 4, 5: Linsen
- 6, 7: Blenden
- 8: Umlenkeinrichtung (Prismenblock)
- 8a: Unterseite der Umlenkeinrichtung
- 8b: Oberseite der Umlenkeinrichtung
- 9: Vergrößerungssystem
- 11: optische Achse
- 12: Achse der Umlenkeinrichtung
- 12a, b, c: Teilstrahlenbündel
- 14, 15, 16: Teilstrahlenbündel
- 16, 17, 18: Umlenkelemente
- 16a, 17a, 18a: Unterkanten der Umlenkelemente
- 16b, 17b, 18b: Oberkante der Umlenkelemente
- 17e: Blech
- 19, 20: verspiegelte Bereiche der Umlenkelemente 17, 18
- 22a, 22b: Beobachtungsstrahlengänge Hauptbeobachter
- 23a, 23b: Beobachtungsstrahlengänge Mitbeobachter
- 24: verspiegelter Bereich des Umlenkelements 16
- 25: Doppelpfeil
- 28, 29: Blenden
- 30a, 30b: keilartige Blöcke
- 100: Mikroskop

## Patentansprüche

1. Beleuchtungseinrichtung für ein wenigstens einen Beobachtungsstrahlengang aufweisendes Operationsmikroskop mit einem Beleuchtungssystem (3, 4, 5, 28, 29) und einer Umlenkeinrichtung (8) zum Umlenken von aus dem Beleuchtungssystem austretendem Licht auf ein zu beobachtendes Objekt, insbesondere ein zu operierendes Auge, wobei die Umlenkeinrichtung, eine Beleuchtung des Objektes unter verschiedenen Beleuchtungswinkeln bezüglich des wenigstens einen Beobachtungsstrahlengangs gestattet, und wobei die Umlenkeinrichtung (8) zwei wenigstens teilweise als physikalische Strahlenteiler ausgebildete Umlenkelemente (16, 17, 18) aufweist,
**dadurch gekennzeichnet, dass** wenigstens eines der Umlenkelemente (17, 18) teilweise vollverspiegelt ausgebildet ist, wobei die Umlenkeinrichtung drei Umlenkelemente (16, 17, 18) aufweist, welche entlang einer optischen Achse (12) positioniert sind, wobei die Umlenkeinrichtung als einheitlicher Prismenblock (8) ausgebildet ist, und die Umlenkelemente (16, 17, 18) als verspiegelte Flächen (19, 20, 24) des Prismenblocks ausgebildet sind.

2. Beleuchtungseinrichtung nach einem der vorstehenden Ansprüche, **dadurch gekennzeichnet, dass** zwei Umlenkelemente derart ausgebildet sind, dass eine gleichzeitige +2°- und - 2°-Beleuchtung des Objektes möglich ist.

3. Operationsmikroskop, insbesondere Stereomikroskop, mit einem Hauptobjektiv (2) und einem diesem nachgeschalteten Vergrößerungssystem (9), **gekennzeichnet durch** eine Beleuchtungseinrichtung nach einem der vorstehenden Ansprüche.

4. Operationsmikroskop nach Anspruch 3, **dadurch gekennzeichnet, dass** es vier Beobachtungskanäle (22a, 22b, 23a, 23b) aufweist.

5. Operationsmikroskop nach einem der Ansprüche 3 oder 4, **gekennzeichnet durch** Blenden (6, 7) zum wahlweisen Ein- und Ausschalten von Beleuchtungsstrahlengängen.

6. Operationsmikroskop nach einem der Ansprüche 3 bis 5, **dadurch gekennzeichnet, dass** die Umlenkeinrichtung (8) bezüglich einer durch Anordnung von Hauptobjektiv (2) und Vergrößerungssystem (9) relativ zueinander definierten optischen Achse des Mikroskops querverschiebbar ist.

## Claims

1. Lighting device for a surgical microscope comprising at least one observation beam path with an illumination system (3, 4, 5, 28, 29) and a deflection device (8) for deflecting light emitted from the illumination system onto an object to be observed, in particular an eye to be operated on, the deflection device providing an illumination of the object under various illumination angles with regard to the at least one observation beam path, the deflection device (8) comprising two deflection elements (16, 17) which are at least partly provided as physical beam splitters,
**characterised in that** at least one of the deflection elements (17, 18) is in part, fully reflective, the deflection device comprising three deflection elements (16, 17, 18) positioned along an optical axis (12), the deflection device being provided as a single-unit prism block (8), and the deflection elements (16, 17, 18) being provided as reflective surfaces (19, 20, 24) of the prism block.

2. Lighting device according to any one of the preceding claims **characterised in that** two deflection elements are provided so as to enable simultaneous +2° and -2° illumination of the object.

3. Surgical microscope, in particular a stereomicroscope, with a main objective (2) and a magnifying system (9) downstream thereof, **characterised by** a lighting device according to any one of the preceding claims.

4. Surgical microscope according to claim 3, **characterised in that** it comprises four observation channels (22a, 22b, 23a, 23b).

5. Surgical microscope according to any one of the claims 3 or 4, **characterised by** shutters (6, 7) for selectively switching observation beam paths on or off.

6. Surgical microscope according to any one of claims 3 to 5, **characterised in that** the deflection device (8) is transversely movable with respect to an optical axis of the microscope defined by the arrangement of the main objective (2) and magnifying system (9) relative to one another.

## Revendications

1. Dispositif d'éclairage pour un microscope d'opération qui présente au moins un parcours pour un faisceau d'observation, comprenant un système d'éclairage (3, 4, 5, 28, 29) et un dispositif de renvoi (8) pour renvoyer la lumière provenant du système d'éclairage vers un objet à observer, en particulier un oeil à opérer, dans lequel le dispositif de renvoi permet un éclairage de l'objet sous différents angles d'éclairement par rapport audit au moins un parcours pour faisceau d'observation, et dans lequel le dispositif de renvoi (8) comprend au moins deux éléments de renvoi (16, 17, 18) réalisés au moins partiellement sous forme de séparateur de faisceau physique,
**caractérisé en ce que** au moins un des éléments de renvoi (17, 18) est réalisé partiellement totalement réfléchissant, le dispositif de renvoi comportant trois éléments de renvoi (16, 17, 18) arrangés le long d'une axe optique (12), le dispositif de renvoi étant réalisé sous forme de bloc à prisme (8) unitaire, et les éléments de renvoi étant realisés tel que faces réfléchissantes (19, 20, 24) du prisme.

2. Dispositif d'éclairage selon l'une des revendications précédentes, **caractérisé en ce que** deux éléments de renvoi sont réalisés de telle manière qu'un éclairage simultané à + 2° et - 2° de l'objet est possible.

3. Microscope d'opération, en particulier stéréomicroscope, comprenant un objectif principal (2) et un système de grossissement (9) installé en aval, **caractérisé par** un dispositif d'éclairage selon l'une des revendications précédentes.

4. Microscope d'opération selon la revendication 3, **caractérisé en ce qu'**il comprend quatre canaux d'observation (22a, 22b, 23a, 23b).

5. Microscope d'opération selon l'une des revendications 3 ou 4, **caractérisé par** des diaphragmes (6, 7) pour mettre sélectivement en service et hors service des parcours de faisceau d'éclairage.

6. Microscope d'opération selon l'une des revendications 3 à 5, **caractérisé en ce que** le dispositif de renvoi (8) est déplaçable transversalement par rapport à un axe optique du microscope défini par l'agencement de l'objectif principal (2) et du système de grossissement (9) l'un par rapport à l'autre.
